# EUROPEAN PATENT APPLICATION

(11) **EP 2 380 559 A1**
(43) Date of publication of application: **26.10.2011**
(21) Application number: 06841714.6
(22) Date of filing: 28.11.2006
(51) Int. Cl.: A61K 9/127

(54) **LIPOSOME PREPARATION METHOD**

(30) Priority: 30.11.2005 ES 200502954
(71) Applicant: ITALFARMACO, S.A., 28108 Alcobendas (ES)
(72) Inventor: MOSCOSO DEL PRADO, Jaime, E-28108 Alcobendas - Madrid (ES); ECHEVARRIA ZAMACONA, Lydia, E-28100 Alcobendas - Madrid (ES)
(74) Representative: de Justo Vàzquez, Mario M.
(86) International application number: PCT/ES2006/000661
(87) International publication number: WO 2007/063156

(57) **Abstract**

The invention relates to a method for preparing liposomes with at least two uniformly-distributed phospholipids, without using organic solvents.

## Description

The invention relates to a method for preparing liposomes which contain at least two phospholipids distributed in a uniform manner in the membranes without using organic solvents.

### BACKGROUND OF THE INVENTION

Liposomes are microscopic vesicles which possess a central aqueous cavity surrounded by a lipid membrane formed by concentric bilayer(s) (lamellas) and are able to incorporate hydrophilic substances (in the aqueous interior) or hydrophobic substances (in the lipid membrane). They can be unilamellar (i.e. have a single lipid bilayer), oligolamellar or multilamellar. The multilamellar vesicles (MLVs) have a size ranging from 0.2 µm to 10 µm, whereas the unilamellar vesicles can be large (LUVs) or small (SUVs) with a size between 0.02 and 0.2 µm.

The most important structural components of liposomes are phospholipids (PLs). The properties of the liposomal vesicles depend, among other factors, on the nature of the constituent. Consequently, if liposomes with certain characteristics are to be obtained, the charge of its polar group and/or the length and the degree of saturation of the chains of its fatty acids must be taken into account.

One important parameter to consider with respect to the formation of liposomes is the rigidity of the lipidic bilayer. The hydrated phospholipid (PL) which forms part of the bilayer may be either in a liquid-crystalline (fluid) or gel state. As the temperature increases, the gel state converts into the liquid-crystalline state. This occurs at a temperature known as the transition temperature (Tc), which is specific to each PL. The Tc is directly proportional to the chain length and inversely proportional to the degree of unsaturation of the fatty acids and depends on the nature of the polar group.

If one wishes to modify the properties of liposomes, it is possible, for example, to incorporate cholesterol (CHOL) and other lipids into the membrane, modify the number of lipidic bilayers, or covalently join natural molecules (for example, proteins, polysaccharides, glycolipids, antibodies, enzymes) or synthetic molecules (polyethyl glycol, for example) to the surface.

Liposomal vesicles are applicable to various fields; for example, they are useful for the administration of pharmaceuticals, cosmetic products, diagnostic agents and genetic material.

There are numerous options for combining phospholipids with an aqueous phase and obtaining liposomes. Depending on the method of preparation and the lipids used, it is possible to obtain vesicles of different sizes, structures and properties.

The step common to the methods for preparing liposomes consists in evaporating the organic solvent in which the lipids are dissolved and then dispersing the lipids in an aqueous solution (buffered or unbuffered). The procedures for preparation differ in terms of the manner in which the lipids are dispersed and can be classified as follows:
a) Hydration of a thin lipidic layer. This is the original method of Bangham et al. Starting with the organic solution of the constituent lipids of the bilayer, a lipidic film is prepared through removal of organic solvent, which can be achieved by means of evaporation (at reduced pressure in a rotary evaporator) or lyophilization. The dry lipidic film deposited on the wall of the flask is hydrated by adding an aqueous solution and agitation at temperatures above the Tc.
b) Reverse-phase evaporation. First a lipidic film is prepared by removing organic solvent. The system is purged with nitrogen and the lipids are redissolved in a second organic phase which is usually constituted by diethyl ether and/or isopropyl ether. The aqueous phase is added to the redissolved lipids. The system is maintained under continuous nitrogen. The gel is formed by removing the second organic solvent.
c) Solvent injection. The lipids dissolved in an organic solvent are injected slowly into a lukewarm aqueous solution.

If the incorporation of more than one PL is desired, care should be taken that said PLs remain homogeneously distributed in the liposomal vesicles. Traditionally, this is achieved by previously dissolving the PLs in an organic solvent and using the resulting organic solvent for preparing the liposomes.

US 4508703 describes a method for obtaining powdery mixtures of at least one amphyphilic lipid and, optionally, at least one component of a hydrophobic or partially hydrophobic nature which includes dissolving the components of the mixture in at least one organic solvent and atomizing the obtained solution into an inert gas. The method permits the preparation of lipidic mixtures which can be easily dispersed in an aqueous medium but does not avoid the use of organic solvents.

WO 92/10166 describes a method for preparing liposomes with an elevated encapsulation capacity. The method permits the use of mixtures of lipids; however, said mixture is obtained by means of previous dissolution of the lipids in an organic solvent and subsequent evaporation. In addition, the contact between lipid(s) and the aqueous solution of active agent is carried out at a temperature above the Tc.

In short, the methods of the prior art for preparing liposomes which contain more than one PL utilize organic solvents for achieving an adequate distribution thereof in the liposomal vesicles. Nonetheless, due to their toxicity and flammability, the organic solvents are reactive and undesirable from an industrial point of view because of their negative repercussions in terms of production costs, safety, work hygiene and the environment.

### SUMMARY OF THE INVENTION

For these reasons, there is a need for a method for preparing liposomes which permits the homogeneous incorporation of at least two PLs in the liposomal membranes without having to prepare a premixture of the PLs dissolved in organic solvents.

The inventors of the present invention found that, by applying an increase in temperature from a temperature below the Tc up to above the Tc to an aqueous suspension of at least two PLs, it is possible to obtain stable liposomes in which the PLs are distributed homogeneously.

Moreover, if the incorporation of at least one active agent is desired, the inventors found that, in starting from aqueous solutions of PLs and increasing the temperature from a temperature below the Tc to a temperature above the Tc, it is possible to prepare liposomes in which the active agent effectively remains encapsulated for the time necessary for its preparation, storage, distribution and utilization.

In accordance with that which is set forth in the foregoing, the present invention relates to a method which permits the preparation of liposomes which contain at least two phospholipids without using organic solvents. This method comprises the following steps:
- adding the phospholipids to an aqueous medium;
- dispersing the resulting suspension by means of vigorous agitation at a temperature below the transition temperature of the mixture of phospholipids for the time necessary to obtain a homogeneous dispersion;
- increasing the temperature until a temperature above the transition temperature of the mixture of phospholipids is reached while maintaining the vigorous agitation until the formation of the liposomes is complete.

Prior to describing particular embodiments, for the purpose of clarification, not limitation, some specific terms will be defined which relate to the primary aspects of the present invention.

"Phospholipid" is understood to be an amphyphilic derivative of glycerol in which one of its hydroxyl groups is esterified with phosphoric acid and the other two hydroxyl groups are esterified with long-chain fatty acids which can be equal to or different from each other and can be saturated or unsaturated. A neutral phospholipid is generally one in which the other phosphoric acid hydroxyl is esterified by an alcohol substituted by a polar group (usually hydroxyl or amino) and whose net charge is zero. A phospholipid with a charge is generally one in which the other phosphoric acid hydroxyl is esterified by an alcohol substituted by a polar group and whose net charge is positive or negative.

"Temperature below the Tc" is understood to be a temperature which is lower than the Tc of the PL having the lowest Tc, and "temperature greater than the Tc" is understood to be a temperature which is greater than the Tc of the PL having the highest Tc.

"aqueous medium" is understood to be a medium which does not contain organic solvents. The aqueous medium is constituted by water and, optionally, solutes such as agents for regulating the pH, substances for regulating osmolarity, and chelating agents.

Encapsulation is understood to be the process of incorporating an active agent into the liposomal vesicles. In the context of the present invention, the encapsulated active agent can remain in the aqueous interior or associated with membranes.

### FIGURES

Fig. 1: Electron microscope photograph of empty liposomes DSPC:DSPG.
Fig. 2: Granulometric analysis at time zero of empty liposomes DSPC:DSPG.
Fig. 3: Z-potential at time zero of empty liposomes DSPC:DSPG.
Fig. 4: Granulometric analysis at time zero of liposomes DSPC:DSPG with 5-FU.
Fig. 5: Z-potential at time zero of liposomes DSPC:DSPG with 5-FU.
Fig. 6: Granulometric analysis at time zero of empty liposomes DSPC:DPPG.
Fig. 7: Z-potential at time zero of empty liposomes DSPC:DPPG.
Fig. 8: Granulometric analysis at time zero of liposomes DSPC:DSPG with diclofenac sodium.
Fig. 9: Z-potential at time zero of liposomes DSPC:DSPG with diclofenac sodium.

### DETAILED DESCRIPTION OF THE INVENTION

In one particular embodiment, the aqueous medium utilized in the initial step of the method of the present invention does not contain an active agent. In this way, it is possible to obtain empty vesicles which are usable as such or in which at least one active agent can then be encapsulated (through active encapsulation). The active encapsulation method used can be any of those known by those skilled in the art such as technical methods using a pH gradient or concentration gradient.

In another particular embodiment, the aqueous medium used in the first step of the method of the present invention contains an active agent. In this way, it is possible to prepare, without additional steps, liposomes with at least one active agent (through passive encapsulation).

In both cases, the active agent to be encapsulated can be added in suspension or solution and, once encapsulated, can remain in the aqueous interior and/or associated with membranes.

The method of the present invention permits encapsulation of active agents such as pharmaceuticals, diagnostic material, cosmetics, foodstuffs, genetic material, etc. in liposomes in an effective and stable manner. Preferably, this method is used to incorporate agents which are pharmaceutically active vis-à-vis liposomes. In principle, any pharmaceutical can be encapsulated using the method of the present invention.

The molar ratio of active substance to PLs will depend on the characteristics of the substance to be encapsulated. The lower limit is determined by the lowest quantity of substance that turns out to be practical for making liposomes given their intended use and can be easily determined by those skilled in the art. The upper limit is a function of the stability of the liposomes.

The liposomes prepared according to the method of the present invention are usually large MLVs with an average diameter between approximately 1 and 10 µm. However, other types of liposomes can also be prepared by selecting other operative parameters.

Optionally, the liposomes obtained using this method can then be subjected to different processing steps using methods which are known to those skilled in the art:
- If a reduction and homogenization of the size distribution is desired, one of the known calibration methods can be applied thereto such as pressurized filtration or extrusion through a microporous membrane.
- They can be subjected to a dehydration process, for example through lyophilization, for their subsequent formulation and/or storage and distribution.
- They can be maintained in suspension in the aqueous solution which does or does not contain active substance (in the former case, a portion of the active agent will be encapsulated and a portion on the exterior of same). The suspensions can be subjected to dilution until the desired concentration of active substance is obtained.
- The unencapsulated (external) active agent can be eliminated using a known method such as diafiltration, centrifugation, gel-filtration.

In principle, any natural, modified, semi-synthetic or synthetic, saturated or unsaturated PL, with a charge or neutral, can be used in the preparation method of the present invention. In addition, any ratio of PLs can be used in the method of this invention.

The liposomes prepared according to the method of the present invention can also contain other lipidic components such as sterols and derivatives (cholesterol, for example); sphingolipids (sphingomyelin, gangliosides, cerebrosides); stearylamine.

PLs and other substances used in the method of the present invention are known by an expert in the field and can be obtained from commercial sources.

### EXAMPLES

The unexpected and advantageous properties of the method of the present invention will become apparent through the following non-limiting examples.

### Example 1: Preparation of "empty" liposomes having the composition distearoylphosphatidylcholine : distearylphosphatidylglycerol (DSPC:DSPG)

A reactor with thermostat is used which is equipped with an anchoring agitation system and a homogenization system of the rotor-stator type. The reactor is turned on and the temperature is set to 53 °C (below the transition temperature of the phospholipids). 360 g of hydrating solution is added to the reactor chamber whose composition (in % p/V) is:
- Na₂HPO₄ 2H₂O 0.07%
- EDTANa₂ 0.1%
- NaH₂PO₄ 2H₂O 0.082%
- NaCl 0.719%

The pH is set to 6.4 ± 0.2. The agitation system is turned on at a speed of 75 rpm. With the agitation system running, 36 g of DSPC and 4 g of DSPG are added. Agitation is continued for 10 minutes.

The homogenization system is turned on at a speed of 6700 rpm until a homogenous dispersion is obtained (5-10 minutes).

The homogenizer is turned off and the agitation system is turned on at 50 rpm. Without stopping agitation, the temperature of the reactor is set to 60 °C and heating is continued until that temperature is reached (in order to ensure that the transition temperature of the phospholipids is exceeded).

Next, the homogenization system is turned on at a speed of 6700 rpm for 6 minutes. The thermostat is turned off.

The liposomal suspension is diluted until a final total weight of 1000 g is reached by adding approximately 600 g of diluent whose composition (in % p/V) is:
- Na₂HPO₄ 2H₂O 0.07%
- EDTANa₂ 0.1%
- NaH₂PO₄ 2H₂O 0.082%
- NaCl 0.719%

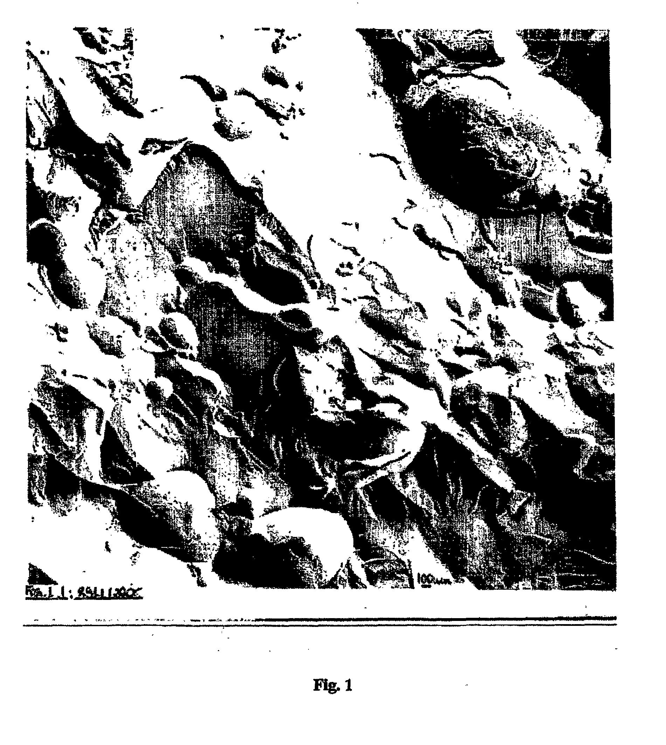

The pH is adjusted to 6.4 ± 0.2. Finally, the agitation system is turned on at 50 rpm for the time necessary to obtain a homogeneous liposomal dispersion. The obtained liposomes are shown in Figure 1.

The size distribution and the z-potential of these liposomes are determined immediately after preparation (time = 0) and at 8 months.

The granulometric analysis of the liposomal suspensions dispersed in aqueous solutions of NaCl 0.9% (p/V) was performed using a laser dispersion method. The equipment used was the Mastersizer 2000 particle analyzer, Hydro 2000G module (Malven Instruments, Worcestershire, United Kingdom).

The zeta potential of the liposomal suspensions dispersed in a phosphate buffer (10 mM, pH 6.2) until a phospholipids concentration of approximately 0.5 x 10⁻³ % (p/V) is reached. The Zetasizer 3000HS (Malven Instruments, Worcestershire, United Kingdom) was used.

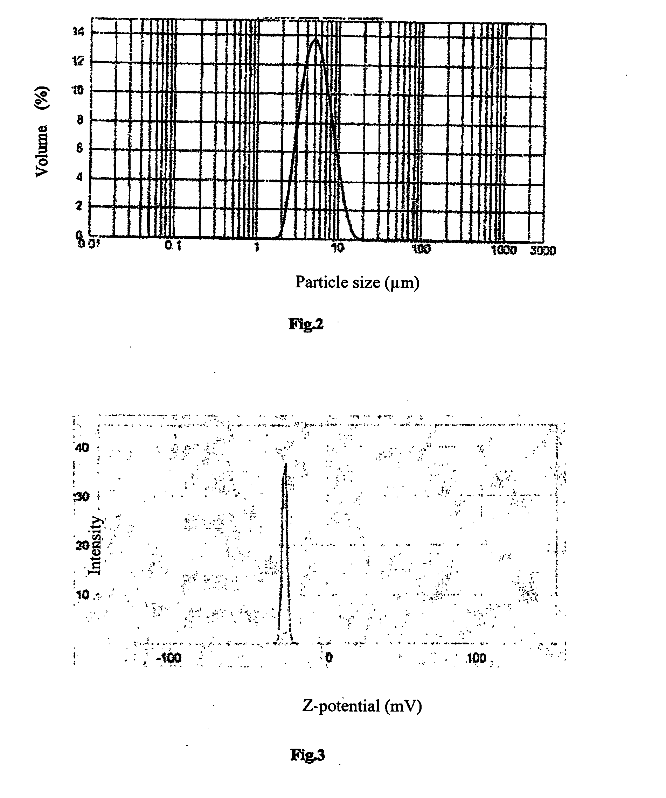

The results are shown in Table 1 and in Figures 2 and 3. These allow for the conclusion that a homogeneous distribution of the PLs is achieved in the liposomal membranes by preparing the vesicles in accordance with the method of the present invention and that this homogeneity is maintained over time.

**Table 1**

| **Time** | **size (µm)** | | | **Number of populations** |
|---|---|---|---|---|
| | **d (0.1)** | **d (0.5)** | **d (0.9)** | |
| **0 months** | 3.100 | 5.198 | 9.176 | 1 |
| **8 months** | 3.091 | 4.958 | 8.952 | 1 |
| | | | | |

| **Time** | **Zeta potential (mV)** | | **Number of** | **populations** |
|---|---|---|---|---|
| **0 months** | -28.1 | | 1 | |
| **8 months** | -29.9 | | 1 | |

### Example 2: Preparation of liposomes having the DSPC:DSPG, with encapsulated fluorouracil (5-FU)

The liposomes are prepared analogously to Example 1 but by adding 72 g of DSPC and 8 g of DSPG to 320 g of hydration solution. In this case, the composition of this solution is:
- Na₂HPO₄ 2H₂O 0.07%
- EDTANa₂ 0.1%
- NaH₂PO₄ 2H₂O 0.082%
- 5-fluorouracil 3%
- NaCl 0.139%

And the composition of the dilution solution is:
- Na₂HPO₄ 2H₂O 0.07%
- EDTANa₂ 0.1%
- NaH₂PO₄ 2H₂O 0.082%
- NaCl 0.477%

Optionally, the external-phase 5-FU can be eliminated using known methods. The size distribution and the z-potential of these liposomes are determined immediately after preparation using the same techniques and equipment as in Example 1.

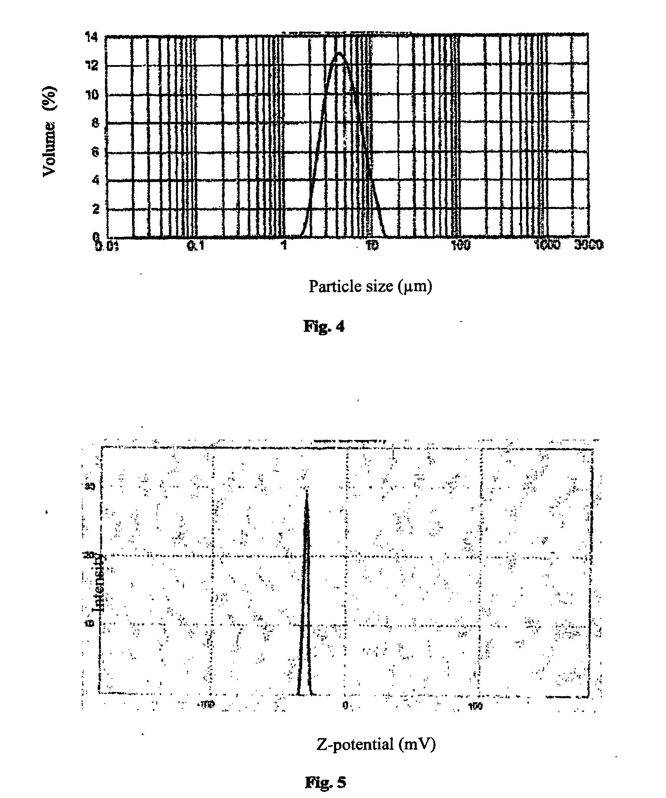

The results are shown in Table 2 and in Figures 4 and 5. Once again, it can be concluded that homogeneous distribution of the membrane PLs is achieved by preparing the liposomes in accordance with the method of the present invention.

**Table 2**

| **Time** | **Size (µm)** | | | **Number of populations** |
|---|---|---|---|---|
| | **d (0.1)** | **d (0.5)** | **d (0.9)** | |
| **0 months** | 2.658 | 4.627 | 8.453 | 1 |
| | | | | |

| **Time** | **Zeta potential (mV)** | | **Number of populations** | |
|---|---|---|---|---|
| **0 months** | -30.2 | | 1 | |

The percentage of encapsulation of 5-FU in the liposomes is determined by means of HPLC using the following chromatographic conditions:

### Equipments: Waters Alliance, column: Hypersil BDS C18 (5 µm), fluid: 1 ml/min, injection volume: 20 µl, mobile phase: 95% ammonium acetate (10 mM, pH 6.8) and 5% acetonitrile.

In order to determine the total quantity of 5-FU (mg/ml), a certain volume of liposomal suspension is taken and is smoothed¹ with methanol until a final phospholipids concentration of 400 µg/ml is reached. This solution is diluted with the mobile phase. It is filtered and injected into the chromatograph.

In order to determine the quantity of free 5-FU, a certain volume of liposomal suspension is taken and is diluted with a solution of NaCl 0.9% (p/V) until a concentration of PLs of 200 µg/ml is reached. The sample is centrifuged for 10 minutes at 13,000 rpm, then the supernatant is filtered and injected into the chromatograph.

The analyzed liposomes have a 5-FU encapsulation of 44.5%.

### Example 3: Preparation of "empty" liposomes having the composition distearoylphosphatidylcholine : dipalmitoylphosphatidylglycerol (DSPC:DPPG)

The liposomes are prepared as in Example 1 with the exception that when the reactor is turned on, the initial temperature is set to 30 °C (with the final temperature being maintained at 60 °C).

The size distribution and the z-potential of these liposomes are determined immediately after preparation using the same techniques and equipment as described in Example 1.

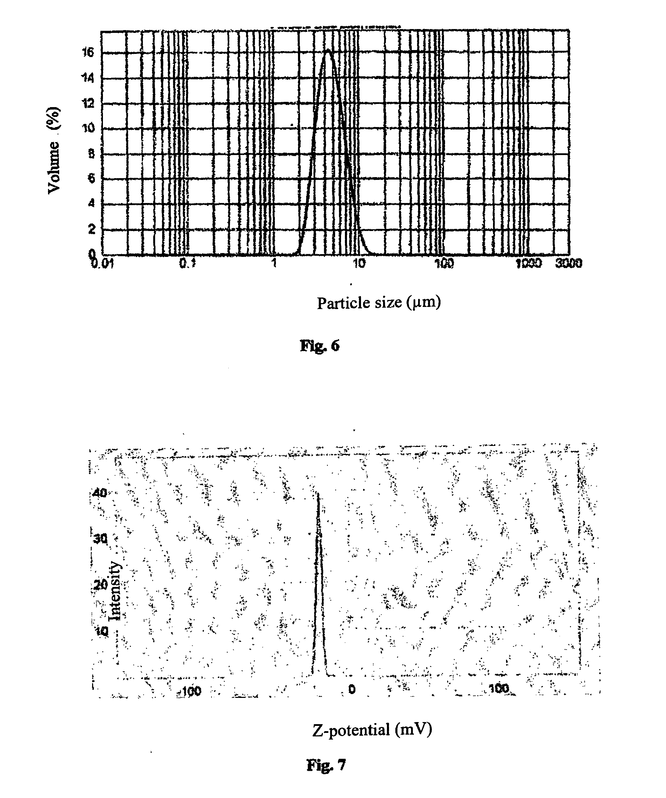

The results are shown in Table 3 and in Figures 6 and 7. Again, it can be concluded that homogeneous distribution of the membrane PLs is achieved by preparing the liposomes in accordance with the method of the present invention.

**Table 3**

| **Time** | **Size (µm)** | | | **Number of populations** |
|---|---|---|---|---|
| | **d (0.1)** | **d (0.5)** | **d (0.9)** | |
| **0 months** | 2.979 | 4.626 | 7.564 | 1 |
| | | | | |

| **Time** | **Zeta potential (mV)** | | **Number of populations** | |
|---|---|---|---|---|
| **0 months** | - 19.4 | | 1 | |

### Example 4: Preparation of liposomes having the composition DSPC:DSPG, with encapsulated diclofenac sodium.

The liposomes are prepared analogously to Example 1 but in using a hydration solution whose composition is:

| | |
|---|---|
| Na₂HPO₄ 2H₂O | 0.07% |
| EDTANa₂0.1% | |
| NaH₂PO₄ 2H₂O | 0.082% |
| NaCl 0.74% | |
| Diclofenac sodium | 0.72% |

And a dilution solution whose composition is:

| | |
|---|---|
| Na₂HPO₄ 2H₂O | 0.07% |
| EDTANa₂0.1% | |
| NaH₂PO₄ 2H₂O | 0.082% |
| NaCl 0.77% | |

The size distribution and the z-potential of these liposomes are determined immediately after preparation and at 4 times using the same techniques and equipment mentioned in Example 1.

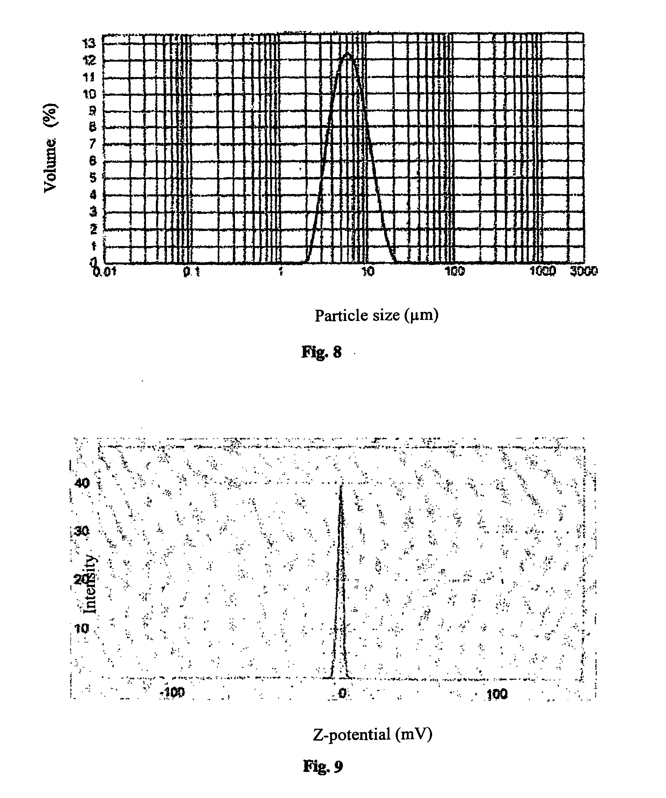

The results are shown in Table 4 and in Figures 8 and 9. These results permit the conclusion that homogeneous distribution of the membrane PLs is achieved by preparing the liposomes in accordance with the method of the present invention and that this homogeneity is maintained over time.

**Table 4**

| **Time** | **Size (µm)** | | | **Number of populations** |
|---|---|---|---|---|
| | **d (0.1)** | **d (0.5)** | **d (0.9)** | |
| **0 months** | 3.440 | 6.202 | 11.374 | 1 |
| **4 months** | 3.490 | 6.362 | 11.529 | |
| | | | | |

| **Time** | **Zeta potential (mV)** | | **Number of populations** | |
|---|---|---|---|---|
| **0 months** | -0.6 | | 1 | |
| **4 months** | -8.1 | | 1 | |

The percentage of encapsulation of diclofenac sodium is determined by means of HPLC immediately after preparation and at 4 months using the following chromatographic conditions:

### Equipment: Waters Alliance, column: LiChrospher 100 RP-18 (5 µm), fluid: 0.9 ml/min, injection volume: 20 µl, mobile phase: 75% methanol and 25% diluted glacial acetic acid (0.3p in 2500p).

In order to determine the total quantity of diclofenac sodium (mg/ml), a certain volume of liposomal suspension is taken and is smoothed² with methanol until a final phospholipids concentration of 200 µg/ml is reached. This solution is diluted with the diluted glacial acetic acid from the mobile phase. It is filtered and injected into the chromatograph.

In order to determine the quantity of free diclofenac sodium, a certain volume of liposomal suspension is taken and is diluted with a solution of NaCl 0.9% (p/V) until a concentration of PLs of 200 µg/ml is reached. The sample is centrifuged for 10 minutes at 13,000 rpm, then the supernatant is filtered and injected into the chromatograph.

The liposomes analyzed at time zero have a diclofenac sodium encapsulation of 80.8%. This very high percentage is likely due to the association of the active ingredient with the phospholipids and, consequently, to an interaction of the diclofenac sodium with the membrane of the liposomes.

The results are shown in Table 5 and permit the conclusion that the encapsulation is maintained over time.

**Table 5**

| **Time** | **Encapsulation (%)** |
|---|---|
| **0 months** | 80.8 |
| **4 months** | 78.9 |

## Claims

1. Method for the preparation of liposomes which comprises:
- adding the phospholipids to an aqueous medium;
- dispersing the resulting suspension by means of vigorous agitation at a temperature below the transition temperature of the mixture of phospholipids for the time necessary to obtain a homogeneous dispersion;
- increasing the temperature until a temperature above the transition temperature of the mixture of phospholipids is reached while maintaining the vigorous agitation until the formation of the liposomes is complete.

2. Method as set forth in claim 1, wherein the aqueous medium contains an active agent.

3. Method as set forth in claim 1, wherein the aqueous medium does not contain an active agent.

4. Method as set forth in claim 3 additionally comprising the encapsulation of an active agent.

5. Method as set forth in claims 2 or 4, wherein the active agent is an pharmacologically active agent.

6. Method as set forth in any of the foregoing claims additionally comprising the application of one of the following processes:
- homogenization and/or reduction of the size of the liposomes;
- dehydration of the liposomal suspension;
- dilution of the liposomal suspension;
- removal of the unencapsulated active agent.
